(19) Europäisches Patentamt
European Patent Office
Office européen des brevets

(11) Publication number: **0 220 339**
**B1**

# (12) EUROPEAN PATENT SPECIFICATION

(45) Date of publication of patent specification: **08.11.89**

(21) Application number: **85115575.4**

(22) Date of filing: **20.12.79**

(60) Publication number of the earlier application in accordance with Art. 76 EPC: **0 013 138**

(51) Int. Cl.[4]: **C 07 D 451/04,**
C 07 D 451/02,
C 07 D 451/14, C 07 D 487/08
// A61K31/395 ,(C07D487/08,
209:00, 209:00),(C07D487/08,
223:00, 209:00)

## (54) Azabicycloalkyl derivatives.

(30) Priority: **30.12.78 GB 7850380**
**15.03.79 GB 7909262**
**09.08.79 GB 7927831**

(43) Date of publication of application:
**06.05.87 Bulletin 87/19**

(45) Publication of the grant of the patent:
**08.11.89 Bulletin 89/45**

(84) Designated Contracting States:
**BE CH DE FR GB IT NL SE**

(56) References cited:
**DE-A-2 748 260**
**GB-A- 774 858**

**CHEMISCHE BERICHTE, vol. 111, no. 5, 1978, pages 1962-1977, Verlag Chemie GmbH, Weinheim, DE; H.-Fr. GRÜTZMACHER et al.: "Untersuchungen zur synchronen oder zweistufigen Fragmentierung der Molekül-Ionen von 3-substituierten Tropanen"**

(73) Proprietor: **BEECHAM GROUP PLC**
**Beecham House Great West Road**
**Brentford Middlesex TW8 9BD (GB)**

(72) Inventor: **Hadley, Michael Stewart**
**9 Coney Gree**
**Sawbridgeworth Hertfordshire (GB)**
Inventor: **King, Francis David**
**8 Heath Row**
**Bishop's Stortford Hertfordshire (GB)**

(74) Representative: **Jones, Pauline et al**
**Beecham Pharmaceuticals Patents & Trade Marks Dept. Great Burgh Yew Tree Bottom Road**
**Epsom Surrey KT18 5XQ (GB)**

(56) References cited:
**JOURNAL OF THE AMERICAN CHEMICAL SOCIETY, vol. 79, pages 4194-4198, Columbus, Ohio, US; S. ARCHER et al.: "3alpha-(2-diethylaminoethyl)-aminotropane and related compounds"**

EP 0 220 339 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel intermediates of use in the preparation of pharmacologically active compounds.

N - (2 - Diethylaminoethyl) - 2 - methoxy - 4 - amino - 5 - chlorobenzamide, 1 - ethyl - 2(methoxy - 5 - sulphamoylbenzamidomethyl)pyrrolidine and N - [4' - (1'' - benzyl) - piperidyl] - 2 - methoxy - 4 - amino - 5 - chlorobenzamide are well known compounds having useful pharmacological activity such as the ability to regulate the gastro-intestinal function, anti-emetic activity and CNS activity.

A certain structurally distinct class of substituted benzamides, which also have useful pharmacological activity, are described in European Patent 13138 (Beecham Group p.l.c.) and certain intermediates used in their preparation are described in European Patent 81054 (Beecham Group p.l.c.).

Chemische Berichte, Vol. 111, No. 5, 1978, pages 1962—1977 and J. Am. Chem. Soc., Vol. 79, pages 4194—4198 (S. Archer et. al.) disclose certain azabicycloalkyl derivatives.

A group of intermediates of formula (XIIIA):

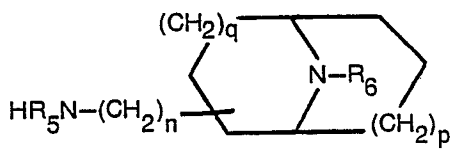

(XIIIA)

wherein

$R_5$ is hydrogen or $C_{1-6}$ alkyl;

$R_6$ is $C_{1-4}$ alkyl; and

n, p and q are independently 0 to 2;

with the proviso that, when n is 0, p is 1 or 2;

is believed to be novel and forms an aspect of the present invention.

Suitable examples of $R_6$ include methyl, ethyl, *n*- and *iso*-propyl and *n*-, *sec*-, *iso*- and *tert*- butyl, particularly methyl, *n*-propyl and *iso*-butyl.

n is preferably 0. q is suitably 0 to 1, preferably 1. p is suitably 0 to 1.

Preferably q is 1 and the $NR_5N\text{-}(CH_2)_n$— moiety is then attached at a position *para* to the N-atom.

European Patent 13138 describes a process for the preparation of a compound of the formula (I) as defined therein, which process comprises reacting an acid of the formula (XII):

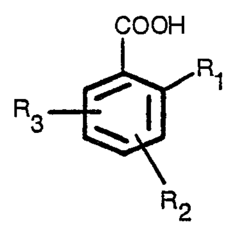

(XII)

or a reactive derivative thereof, with an intermediate compound of formula (XIIIA) as hereinbefore defined; and thereafter if desired or necessary converting a group $R_2$ or $R_3$ in the thus formed compound to another group $R_2$ or $R_3$ respectively; and optionally forming a pharmaceutically acceptable salt of the resultant compound of the formula (I).

It will be realised that in the compounds of the formula (I) the $—CO—NR_5—(CH_2)_n—$ linkage may have an α or β orientation with respect to the ring of the bicyclic moiety to which it is attached. A mixture of α and β isomers of the compound of the formula (I) may be synthesised nonstereospecifically and the desired isomer separated conventionally therefrom, e.g. by chromatography; or alternatively the α or β isomer may if desired be synthesised from the corresponding α or β form of the compound of the formula (XIIIA).

Synthesis from the corresponding α or β isomer of the compound of the formula (XIIIA) is in general preferred.

The α or β form of a compound of formula (XIIIA) may be prepared by known stereospecific processes, such as those leading to the α or β isomer of the compound of formula (XIIIA) depicted in Scheme 1 of EP 81054.

The precursor of a compound of formula (XIII) may be stereospecifically synthesised, such as *via* the azide, and then converted to the corresponding desired isomer of the compound of formula (XIIIA) under non-stereospecific conditions with retention of configuration. Alternatively, the precursor may itself have no asymmetric centre at the relevant position, such as oximes and imines, but may be converted under stereospecific conditions to the desired isomer of the compound of formula (XIIIA).

Alternatively, a mixture of the α or β isomers of a compound of formula (XIIIA) may be synthesised

non-stereospecifically and the desired isomer separated conventionally therefrom e.g. by chromatography.

However, in this case it is generally more convenient to react the mixture to give a mixture of α and β isomers of the compound of formula (I) and to separate these if desired as hereinbefore described.

Schemes 1 and 2 of EP 81054 illustrate stereospecific and non-stereospecific synthetic routes to the intermediates of formula (XIIIA) wherein n is 0 and wherein n is 1 or 2, respectively.

The following Examples illustrate the preparation of the intermediates of the invention.

Nomenclature note: Tropane is 8-methyl-8-azabicyclo[3.2.1]octane and derivatives thereof are named accordingly in the following.

N.B. Compound Numbers refer to the compounds of the Examples described in EP 13138.

DESCRIPTION

3β-amino-8-methyl-8-azabicyclo[3.2.1]octane (D8), intermediate for Compound 19

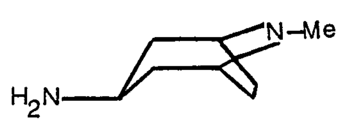

(D8)

(a) Tropinone oxime (D7)

Tropinone (3.68 g; 0.0265 mole) was dissolved in ethanol (50 ml) containing pyridine (4—5 ml) and treated with hydroxylamine hydrochloride (1.90 g). The mixture was heated under reflux for 30 minutes, cooled, treated with solid potassium carbonate (ca. 10 g) and water (ca. 5 ml). The ethanol was removed *in vacuo* and the mixture extracted with chloroform (3 × 150 ml). The combined extracts were dried ($K_2CO_3$), filtered and evaporated *in vacuo*. The resulting solid was recrystallised from ethyl acetate/petrol ether 40—60 to yield tropinone oxime (3.1 g; 76%) as colorless crystals m.pt. 114—115°C.

(b) 3β-amino-8-methyl-8-azabicyclo[3.2.1]octane (D8)

Tropinone oxime (3.08 g; 0.02 mole) was dissolved in anhydrous amyl alcohol (100 ml) and heated to almost boiling. Sodium (ca. 3.0 g) was added portionwise over 1 hour then the mixture left to cool overnight. The mixture was treated with 5N hydrochloric acid (ca. 80 ml), and extracted with ethyl acetate (3 × 150 ml). The acidic aqueous layer was separated, basified with sodium hydroxide and re-extracted with ethyl acetate (4 × 150 ml) and the combined extracts were dried ($K_2CO_3$) filtered and evaporated *in vacuo* to yield (D8) (2.25 g; 80%) as a colourless oil, used without further purification.

Example 1

The following intermediate of the general formula

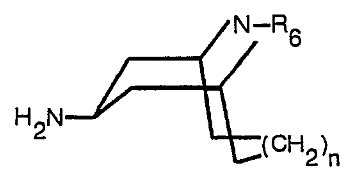

was prepared analogously to the compound of the Description.

| Intermediate No. | $R_6$ | n | For compound | Yield % |
|---|---|---|---|---|
| D16 | Me | 1 | 22 | 60 |

Example 2

3-Amino-9-methyl-9-azabicyclo[3.3.1]nonane (D15); intermediate for Compound 21

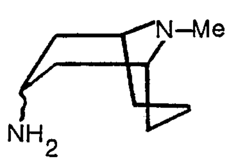

(D25)

N-Methyl-9-azabicyclo[3.3.1]nonan-3-one oxime (3.25 g, 0.02 mole) was dissolved in ethanol and

hydrogenated over Raney nickel in the presence of ammonium acetate at 300 p.s.i. 2.02 MPa at 50°C for 24 hours. The mixture was filtered, evaporated in vacuo, dissolved in dilute hydrochloric acid, basified and extracted into ethyl acetate. The combined organic layers were dried ($K_2CO_3$), filtered and evaporated *in vacuo* to yield 3-amino-9-methyl-9-azabicyclo[3.3.1]nonane (2.67 g, 90%), used without further purification.

The product was a single diastereomer believed to be the α-isomer; which is *endo*-9-methyl-9-azabicyclo[3.3.1]nonan-3-amine.

Example 3

2-aminomethyl-8-methyl-8-azabicyclo[3.2.1]octane (D31); intermediate for Compound 37

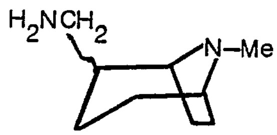

(D31)

including all isomeric forms

Methyl isocyanide (5.04 g, 0.026 mol) was added to (±)-tropan-2-one (2 g, 0.143 mol) in dimethoxyethane (80 ml) and the solution cooled to 0°C. Ethanol (2 ml) was added followed by addition of potassium *tert*-butoxide (5.64 g, 6.05 mol). The mixture was then heated at 50°C for three hours, cooled and poured into a saturated potassium carbonate solution (300 ml). This was extracted with ethyl acetate (3 × 100 ml) and the combined extracts dried ($K_2CO_3$), filtered and evaporated to give a crude oil which was absorbed on alumina (40 g, grade 1 neutralised by addition of 10% water) from ethereal solution. The solution was eluted with a progressively graded mixture of ether, ethyl acetate and methanol to give 8-methyl-8-azabicyclo[3.2.1]octane-2-nitrile (1.1 g — 46%) as an oil.

The nitrile (1.1 g) in tetrahydrofuran (20 ml) was added to lithium aluminium hydride (0.5 g) in tetrahydrofuran (30 ml) and the mixture stirred for three hours. Water (0.5 ml), sodium hydroxide solution (10%) (0.75 ml) and water (1.25 ml) were added successively and the mixture filtered. The filtrate was dried ($K_2CO_3$) and evaporated to give crude (D31) (1 g) as an oil.

The product is a racemate of a single diastereomer, believed to be the (±)-α-isomer.

**Claims**

1. A compound of formula (XIIIA):

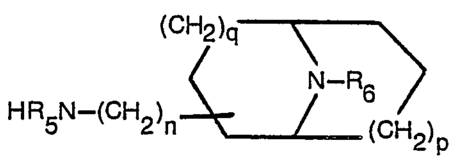

(XIIIA)

wherein

$R_5$ is hydrogen or $C_{1-6}$ alkyl;

$R_6$ is $C_{1-4}$ alkyl; and

n, p and q are independently 0 to 2;

with the proviso that, when n is 0, p is 1 or 2.

2. A compound according to claim 1, wherein $R_5$ is hydrogen.

3. A compound according to either of the preceding claims, wherein n is 0.

4. A compound according to any one of the preceding claims, wherein p is 0 or 1.

5. A compound according to any one of the preceding claims, wherein q is 1.

6. A compound according to claim 5, wherein the $HR_5N\text{-}(CH_2)_n$ moiety is attached to the bicyclic ring in the position para to the N—$R_6$ moiety.

7. A compound according to claim 6, wherein the $HR_5N(CH_2)_n$ moiety has an α-orientation with respect to the bicyclic ring to which it is attached.

8. A compound according to any one of the preceding claims wherein $R_6$ is methyl or ethyl.

9. endo-9-Methyl-9-azabicyclo[3.3.1]nonan-3-amine.

**Patentansprüche**

1. Eine Verbindung der Formel (XIIIA)

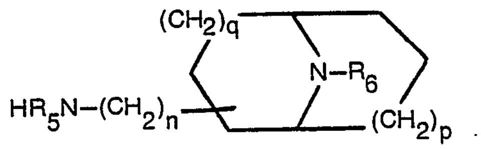

(XIIIA)

in welcher

$R_5$ Wasserstoff oder $C_{1-6}$ Alkyl ist;

$R_6$ $C_{1-4}$-Alkyl ist; und

n, p und q jeweils unabhängig voneinander den Wert 0 bis 2 haben mit der Maßgabe, daß, wenn n den Wert 0 hat, p den Wert 1 oder 2 hat.

2. Eine Verbindung gemäß Anspruch 1, in welcher $R_5$ Wasserstoff ist.

3. Eine Verbindung gemäß jedem der vorhergehenden Ansprüche, in welcher n den Wert 0 hat.

4. Eine Verbindung gemäß irgendeinem der verhergehenden Ansprüche, in welcher p den Wert 0 oder 1 hat.

5. Eine Verbindung gemäß irgendeinem der verhergehenden Ansprüche, in welcher q den Wert 1 hat.

6. Eine Verbindung gemäß Anspruch 5, in welcher der Molekülteil $HR_5N—(CH_2)_n$ in p-Stellung zu dem Molekülteil $N—R_6$ an den bicyclischen Ring geknüpft ist.

7. Eine Verbindung gemäß Anspruch 6, in welcher der Molekülteil $HR_5N—(CH_2)_n$ eine α-Orientierung in bezug auf den bicyclischen Ring aufweist, an den er geknüpft ist.

8. Eine Verbindung gemäß irgendeinem der vorhergehenden Ansprüche, in welcher $R_6$ Methyl oder Äthyl ist.

9. endo-9-Methyl-9-azabicyclo[3.3.1]nonan-3-amin.

**Revendications**

1. Composé, caractérisé par la formule (XIIIA)

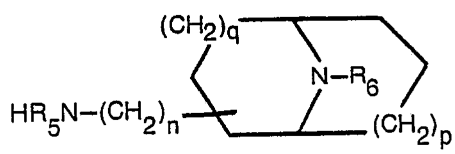

(XIIIA)

dans laquelle

$R_5$ est un atome d'hydrogène ou un groupe alkyle en $C_{1-6}$;

$R_6$ est un groupe alkyle en $C_{1-4}$; et

n, p et q sont indépendamment 0 à 2;

à la condition que dans la cas où n est égal à zéro, p soit 1 ou 2.

2. Composé suivant la revendication 1, caractérisé en ce que $R_5$ est un atome d'hydrogène.

3. Composé suivant l'une quelconque des revendications précédentes, caractérisé en ce que n est égal à zéro.

4. Composé suivant l'une quelconque des revendications précédentes, caractérisé en ce que p est égal à zéro ou un.

5. Composé suivant l'une quelconque des revendications précédentes, caractérisé en ce que q est égal à un.

6. Composé suivant la revendication 5, caractérisé en ce que la partie $HR_5N—(CH_2)_n$ est fixée au noyeu bicyclique en position para par rapport à la partie $N—R_6$.

7. Composé suivant la revendication 6, caractérisé en ce que la partie $HR_5N—(CH_2)_n$ a une orientation α par rapport au noyau bicyclique auquel elle est attachée.

8. Composé suivant l'une quelconque des revendications précédentes, caractérisé en ce que $R_6$ est un groupe méthyle ou éthyle.

9. Composé suivant la revendication 1, caractérisé en ce qu'il s'agit de la endo-9-méthyl-9-azabicyclo-[3,3,1]nonan-3-amine.